(19) Europäisches Patentamt
European Patent Office
Office européen des brevets

(11) Veröffentlichungsnummer: **0 001 251**
**A1**

(12) # EUROPÄISCHE PATENTANMELDUNG

(21) Anmeldenummer: **78100864.4**

(22) Anmeldetag: **11.09.78**

(51) Int. Cl.$^2$: **C 07 C 126/00**

(30) Priorität: **19.09.77 DE 2742158**

(43) Veröffentlichungstag der Anmeldung:
**04.04.79 Patentblatt 79/7**

(84) Benannte Vertragsstaaten:
**BE CH FR GB LU NL SE**

(71) Anmelder: **SKW Trostberg Aktiengesellschaft**
**Dr.-Albert-Frank-Strasse 32 Postfach 1150/1160**
**D-8223 Trostberg(DE)**

(72) Erfinder: **Hintermaier, Helmut, Dr.**
**Mühlbachweg 20**
**D-8223 Trostberg(DE)**

(72) Erfinder: **Michaud, Horst, Dr.**
**Sonnenleite 11**
**D-8223 Trostberg(DE)**

(72) Erfinder: **Obinger, Matthias**
**Ludwig-Thoma-Strasse 6**
**D-8223 Trostberg(DE)**

(74) Vertreter: **Weickmann, Heinrich, Dipl.-Ing. et al,**
**Postfach 860 820 Möhlstrasse 22**
**D-8000 München 86(DE)**

(54) **Herstellung substituierter Harnstoffe.**

(57) Verfahren zur Herstellung von Harnstoffderivaten der allgemeinen Formel

$$R_1R_2N-\underset{\underset{O}{\|}}{C}-NHR_3$$

wobei $R_1$, $R_2$ und $R_3$ gleiche oder verschiedene niedere Alkylgruppen, die gegebenenfalls durch weitere Reste wie Äther- oder Arylgruppen, einen heterocylischen Rest oder Chlor substituiert sein können, bedeuten, $R_1$ und $R_3$ jeweils eine Alkylgruppe und $R_2$ Wasserstoff darstellt oder $R_1$ und $R_2$ Alkylgruppen sind, die gegebenenfalls durch Wasserstoff ersetzt sein können, wenn $R_3$ eine Arylgruppe, gegebenenfalls durch eine Hydroxy-, Chlor-, Methoxy- oder Alkylgruppe substituiert, ist aus Kohlenoxisulfid und Aminen.

EP 0 001 251 A1

# Herstellung substituierter Harnstoffe

Die Erfindung betrifft die Herstellung substituierter Harnstoffderivate aus Kohlenoxisulfid und Aminen.

Es ist bekannt, Harnstoff und substituierte Harnstoffe aus Kohlenmonoxid, Schwefel und Ammoniak bzw. primären Monoaminen darzustellen. Die Reaktion verläuft bei 70 - 160°C und einem Druck von 10,5 - 17,5 kg/cm² (DT-PS 10 64 944).

Die DT-PS 12 32 572 beschreibt die Herstellung von Harnstoff bzw. aliphatisch substituierten Harnstoffen aus Kohlenoxisulfid und Ammoniak oder aliphatischen Aminen unter Druck bei erhöhter Temperatur.

Bekannt ist ferner die Umsetzung von Kohlenoxisulfid mit araliphatischen und aromatischen Aminen (Chem. Berichte 83 (1950), S. 258 - 261). Danach reagieren rein aromatische Amine nicht mit Kohlenoxisulfid in der Kälte bei Normaldruck. Auch scharfe Reaktionsbedingungen führen nur zu geringen Ausbeuten an symmetrischen Arylharnstoffen. So entsteht z.B. Diphenylharnstoff bei 6-stündiger Autoklavenbehandlung (150°C) von Kohlenoxisulfid und Anilin nur in einer Menge von 0,6 %.

Allen bekannten Herstellungsarten gemeinsam ist das Arbeiten unter erhöhtem Druck, was bei einer technischen Produktion stets mit zusätzlichen Kosten und Risiken behaftet ist.

Es bestand daher die Aufgabe, diesen Nachteil der oben beschriebenen Verfahren zu überwinden, ferner einen Syntheseweg für die bisher nicht zugänglichen, durch Alkyl- und Arylreste substituierten Harnstoffe aus Kohlenoxisulfid und den entsprechenden Aminen zu ermöglichen.

Es ist nun gelungen, Harnstoffderivate der allgemeinen Formel

$$R^1R^2N - \underset{\underset{O}{\|}}{C} - NHR^3$$

darzustellen, in der $R^1$, $R^2$ und $R^3$ gleiche oder verschiedene niedrigmolekulare Alkylgruppen, die gegebenenfalls durch weitere Reste wie Äther- oder Arylgruppen, einen heterocyclischen Rest oder Chlor substituiert sein können, bedeuten, $R^1$ und $R^3$ jeweils eine Alkylgruppe und $R^2$ Wasserstoff darstellt oder $R^1$ und $R^2$ Alkylgruppen sind, die gegebenenfalls durch Wasserstoff ersetzt werden können, wenn $R^3$ eine gegebenenfalls durch eine Hydroxy-, Chlor-, Methoxy- oder Alkylgruppe substituierte Arylgruppe ist, aus Kohlenoxisulfid und Aminen der allgemeinen Formel

$$HN(R^1R^2)$$

in der $R^1$ und/oder $R^2$ auch $R^3$ sein und die angegebene Bedeutung haben, dadurch, daß man die Reaktionspartner etwa in molekularem Verhältnis unmittelbar in einem einzigen Reaktionsschritt umsetzt.

Das erfindungsgemäße Verfahren eignet sich daher zur Synthese aliphatisch substituierter Harnstoffe, aliphatischaromatisch sowie aromatisch-aromatisch substituierter Harnstoffe.

Im Falle der aliphatischen Derivate reagiert Kohlenoxisulfid mit Dialkylamin zum Dialkylammoniumsalz der N-Dialkylthiocarbaminsäure, wobei das entstandene Salz

isoliert und destillativ gereinigt wird, da es sich entgegen der Erwartung unter Ausschluß von Luftsauerstoff als überraschend beständig erwies. Vorzugsweise setzt man jedoch die entstandene Verbindung gleich ohne Isolation und gesonderte Reinigung in einem zweiten Schritt mit dem betreffenden aromatischen oder aliphatischen Amin unter Abspaltung von Dialkylammoniumhydrogensulfid oder Dialkylammoniumsulfid zum entsprechenden Harnstoff um. Aus dem Dialkylammoniumhydrogensulfid gewinnt man durc die Umsetzung mit Basen (Alkalihydroxide, $Na_2CO_3$, $Ca(OH)_2$) oder eine Ionenaustauscherbehandlung wieder das freie Amin zurück.

Die entsprechende Gleichung, der Einfachheit halber mit Dimethylamin formuliert, lautet:

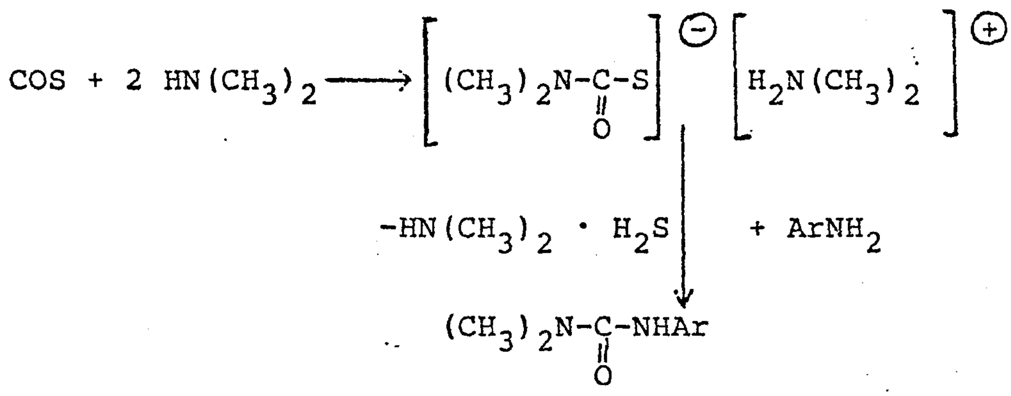


Die besonders interessierenden N,N-Dialkyl- und N'-Arylharnstoffe entstehen durch Einwirkung des Arylamins auf das Dialkylammoniumsalz der N,N-Dialkylthiocarbaminsäure, das entsprechend vorstehender Gleichung aus Kohlenoxisulfid und Dialkylamin erhalten wurde. Das Arylamin kann dabei sowohl vor dem Einleiten von Kohlenoxisulfid als auch nacher zugesetzt werden. Kohlenoxisulfid, Dimethylamin und das Arylamin werden vorzugsweise etwa im Molverhältnis 1:1:1 eingesetzt. Der Reaktionsablauf läßt sich durch folgendes Reaktionsschema wiedergeben.

$$COS + 2HN(CH_3)_2 \rightleftharpoons \left[(CH_3)_2N-\underset{\underset{O}{\|}}{C}-S\right]^{\ominus} \left[H_2N(CH_3)_2\right]^{\oplus}$$

$$\left[(CH_3)_2N-\underset{\underset{O}{\|}}{C}-S\right]^{\ominus} \left[H_2N(CH_3)_2\right]^{\oplus} + ArNH_2 \rightleftharpoons \left[(CH_3)_2N-\underset{\underset{O}{\|}}{C}-S\right]^{\ominus} \left[H_3NAr\right]^{\oplus} + HN(CH_3)_2$$

$$\left[(CH_3)_2N-\underset{\underset{O}{\|}}{C}-S\right]^{\ominus} \left[H_3NAr\right]^{\oplus} \longrightarrow (CH_3)_2N-\underset{\underset{O}{\|}}{C}-\underset{\underset{H}{|}}{N}-Ar + H_2S$$

Völlig überraschend werden dabei keine symmetrischen Nebenprodukte wie N,N-Dialkyl-N',N'-dialkylharnstoff oder N-Aryl-N'-arylharnstoff gebildet. Selbst, wenn Alkylamin und Arylamin nebeneinander vorliegen, erhält man bei geeigneter Reaktionsführung chromatografisch reinen, durch den Alkyl- und Arylrest in N,N'-Stellung disubstituierten Harnstoff.

Der Reaktionsablauf läßt sich daher auch durch nachfolgende vereinfachte Summengleichung beschreiben:

$$COS + HN(CH_3)_2 + ArNH_2 \longrightarrow (CH_3)_2N-\underset{\underset{O}{\|}}{C}-NHAr + H_2S$$

Die N,N'-Diarylharnstoffe werden unter Einleiten von COS aus dem N,N-Dimethyl-N'-aryl-harnstoff gemäß der Reaktion

$$Ar-\underset{\underset{H}{|}}{N}-\underset{\underset{O}{\|}}{C}-N(CH_3)_2 + ArNH_2 \longrightarrow Ar-NH-\underset{\underset{O}{\|}}{C}-NHAr + (CH_3)_2NH$$

hergestellt. Der N,N-Dimethyl-N'-arylharnstoff, der u.U. nach Beispiel 6 entsteht, braucht dazu nicht isoliert zu werden.

Kohlenoxisulfid als eine der Ausgangsverbindungen ist ent-

sprechend der DAS 12 22 023 einfach aus Kohlenmonoxid und Schwefel herzustellen (I). Nachdem auch der während der Reaktion II anfallende Schwefelwasserstoff durch geeignete Verfahren, z.B. den Claus-Prozeß, wieder zu Schwefel oxidiert werden kann (III), läßt sich die erfindungsgemäße Harnstoffsynthese auf die einfache Gleichung IV zurückführen:

$$\text{I} \quad CO + S \longrightarrow COS$$

$$\text{II} \quad COS + 2\,HNR_2 \longrightarrow NR_2-\underset{\underset{O}{\|}}{C}-NR_2 + H_2S$$

$$\text{III} \quad H_2S + 1/2\,O_2 \longrightarrow H_2O + S$$

---

$$\text{IV} \quad CO + 2\,HNR_2 + 1/2\,O_2 \longrightarrow NR_2-\underset{\underset{O}{\|}}{C}-NR_2 + H_2O$$

Damit tritt keine reaktionsbedingte Salzbelastung des Abwassers auf.

Geeignete Alkylamine sind alle kurzkettigen Amine wie Dimethylamin, Diäthylamin etc. Als Arylamine können verwendet werden: Anilin, Naphtylamin, p-Chloranilin u.a. Selbst schwach basische Amine (z.B. 3,5-Dichloranilin) oder sterisch stark gehinderte (z.B. 2,6-Di-isopropylanilin) ergeben bei Anwendung des erfindungsgemäßen Verfahrens relativ hohe Ausbeuten an der gewünschten Harnstoffverbindung.

Die Umsetzung kann sowohl in Lösungsmitteln wie Dekalin, Benzol, Chlorbenzol, Xylol, höheren Alkoholen (von Butanol homolog ansteigend), Methylbutylketon oder vorzugsweise Toluol als auch lösungsmittelfrei in der Schmelze durchgeführt werden. Dazu geht man entweder vom Dialkylammonium-dialkylcarbaminat aus oder vereinigt Kohlenoxisulfid und das entsprechende Amin in geeigneten Reaktionsgefäßen. Lösungsmittel mit funktionellen Gruppen, die mit

Kohlenoxisulfid reagieren, sind, wenigstens in größerer Menge, zu vermeiden.

Die nach dem erfindungsgemäßen Verfahren darstellbaren substituierten Harnstoffe finden Verwendung als wertvolle Zwischen- oder Endprodukte für biozid wirkende Präparate, für Polymere, Pharmazeutika etc.

Die nachfolgenden Beispiele sollen die Erfindung erläutern, jedoch nicht einengen.

Beispiel 1a

N,N-Dimethyl-N'-phenylharnstoff

In einem Dreihalskolben mit Rührer, Rückflußkühler, Doppeleinleitungsrohr (inneres Rohr für Dimethylamin, äusseres Rohr für Kohlenoxisulfid) wurden 50 ml Toluol und 0,0333 Mol Anilin vorgelegt und während 55 min 0,0314 Mol Dimethylamin sowie ca. 3 g Kohlenoxisulfid eingeleitet. Die Lösung wurde dabei von 20°C auf 82°C erhitzt (77°C nach 10 min). Anschließend wurden bei 82 bis 99°C während 245 min weitere 3 g Kohlenoxisulfid eingeleitet. Die Reaktionslösung wurde abgekühlt, filtriert, der Filterkuchen mit wenig Toluol gewaschen und bei 60°C im Vakuum getrocknet.
Ausbeute: 8 g (81,2 %) kristallines Produkt.

N = 17,12 % (Th. N = 17,06); Fp. 132 - 134°C

Durch Wiederverwendung der Mutterlauge im folgenden Ansatz ließ sich bei einmaligem Wiedereinsatz die Durchschnittsausbeute auf 93 % steigern, wobei die Reinheit des Produktes unverändert blieb.

Beispiel 1b

N,N-Dimethyl-N'-phenylharnstoff

Einwaage: 0,05 Mol DMA-Salz
0,09 Mol Anilin
Lösungsmittel: Toluol

| $H_2O$ Zusatz | Reaktion Minuten | Temp. °C max. | Ausbeute % |
|---|---|---|---|
| 0,5 % | 240 | 103 | 71,49 |
| 1,0 % | 240 | 101 | 70,03 |
| 3,0 % | 240 | 100 | 58,83 |
| 4,0 % | 240 | 98 | 51,49 |
| 6,0 % | 240 | 86 | 44,09 |
| 10,0 % | 240 | 73 | 14,49 |

Beispiel 2

N,N-Dimethyl-N'-p-chlorphenylharnstoff (Monuron)

In der unter 1a beschriebenen Apparatur wurden 100 ml Toluol und 0,068 Mol p-Chloranilin vorgelegt und während 55 min 0,068 Mol Dimethylamin und Kohlenoxisulfid in solchem Strome eingeleitet, daß während der Gesamtreaktionszeit von 220 min insgesamt 8,3 g Kohlenoxisulfid verbraucht wurden. Man erhitzte von 40°C auf 97°C Endtemperatur (80°C nach 10 min), kühlte und filtrierte das ausgefallene Produkt ab, das man im Vakuum trocknete. Das Filtrat wurde in weiteren Ansätzen als Reaktionsmedium wiederverwendet.

Ergebnis:

| Lösungsmittel | Reaktion Endtemp. (°C) | Zeit (min) | % N (14,10) | % Cl (17,85) | Ausbeute % |
|---|---|---|---|---|---|
| Toluol | 97 | 220 | 13,94 | 17,90 | 61,4 |
| Filtrat | 98 | 270 | 14,02 | 18,10 | 87,5 |
| Filtrat | 95 | 195 | 14,03 | 17,99 | 91,5 |
| Filtrat | 98 | 270 | 14,15 | 17,50 | 97,4 |

Die Durchschnittsausbeute betrug 84,4 %.

Beispiel 3

N,N-Dimethyl-N'-3,4-dichlorphenylharnstoff (Diuron)

In der beschriebenen Apparatur wurden 100 ml Toluol und 0,03 Mol 3,4-Dichloranilin vorgelegt, während 30 min 0,029 Mol Dimethylamin und im Verlauf von 260 min 7 g (0,117 Mol) Kohlenoxisulfid eingeleitet. Die Lösung wurde dabei von 20°C auf 91°C erhitzt (77°C nach 10 min).

Der Ansatz wurde abgekühlt, filtriert und der Filterkuchen bei 60°C im Vakuum getrocknet. In den folgenden Ansätzen wurde jeweils das Filtrat des vorhergehenden Ansatzes wiederverwendet.

Ergebnis:

| Lösungsmittel | Reaktion Endtemp. (°C) | Zeit (min) | % N (12,02) | % Cl (30,42) | Ausbeute % |
|---|---|---|---|---|---|
| Toluol | 91 | 260 | 12,26 | 30,40 | 21,0 |
| Filtrat | 89 | 330 | 12,06 | 30,15 | 90,7 |
| Filtrat | 95 | 300 | 12,06 | 30,10 | 91,7 |
| Filtrat | 97 | 330 | 12,10 | 30,40 | 94,5 |
| Filtrat | 92 | 300 | 12,10 | 30,10 | 157,0 |

Die Durchschnittsausbeute betrug 91,1 %.

Beispiel 4

N,N-Methylbutyl-N'-3,4-dichlorphenylharnstoff (Neburon)

Vorgelegt wurden 50 ml Toluol mit 4,05 g (0,025 Mol) 3,4-Dichloranilin und während 65 min 2,18 g (0,025 Mol) N-Methyl-N-butylamin zugetropft. Im Verlauf der Reaktion (260 min) wurden 9 g Kohlenoxisulfid eingeleitet. Die Temperatur wurde dabei von 60 auf 90°C angehoben. Nach dem Abkühlen der Lösung wurde diese filtriert und das Filtrat wieder eingesetzt.

10 ml Filtrat, 2,18 g (0,025 Mol) Methylbutylamin wurden in einem Tropftrichter und das restliche Filtrat mit 4,05 g (0,025 Mol 3,4-Dichloranilin in einem Kolben vorgelegt. Kohlenoxisulfid wurde in solcher Stärke erst durch den Tropftrichter, dann durch den Kolbeninhalt geleitet, daß die Reaktion während 270 min ca. 9 g Kohlenoxisulfid verbrauchte. Der Kolbeninhalt wurde anschließend 30 min auf 80°C erhitzt und im Verlauf von 120 min die Reaktionslösung aus dem Tropftrichter zugetropft. Nach nochmaligem Einleiten von Kohlenoxisulfid bei gleichzeitigem Rühren entstanden noch die Produkte der nachfolgenden Zusammenstellung.

| Medium | Reaktion | | % N | % Cl | Ausbeute |
|---|---|---|---|---|---|
| | Endtemp. (°C) | Zeit (min) | (10,18) | (25,77) | |
| Toluol | 90 | 260 | 10,34 | 25,80 | 11,6 |
| Filtrat | 96 | 270 | 10,45 | 25,90 | 81,4 |
| Filtrat | 106 | 360 | 10,39 | 25,60 | 106,1 |
| Filtrat | 107 | 330 | 10,14 | 26,40 | 102,6 |
| Filtrat | 106 | 330 | 10,43 | 25,70 | 98,8 |
| Filtrat | 105 | 330 | 10,46 | 25,70 | 94,5 |

Die Durchschnittsausbeute betrug 82,5 %.

Beispiel 5

Herstellung des Dimethylammoniumsalzes der N-Dimethylthiocarbaminsäure

45,4 g (1,0 Mol) Dimethylamin wurden bei -5°C durch Einleiten in 100 ml Toluol gelöst und anschließend bei -5°C bis +20°C 32,0 g (0,533 Mol) Kohlenoxisulfid eingeleitet. Die gewünschte Verbindung fiel in schönen weißen Kristallen aus. Das Salz wurde abfiltriert, mit Äther gewaschen und im Ölpumpenvakuum bei Raumtemperatur getrocknet. Ausbeute 68 g (91 %), Reinheit 99,6 %.

Beispiel 6

N,N-Dimethyl-N'-phenylharnstoff

3,78 g 99,6 %iges Dimethylammoniumsalz der Dimethylthiocarbaminsäure (DADTC), 2,33 g Anilin und 50 ml Dekalin wurden 215 min lang unter Rühren auf max. 108°C erhitzt, wobei sich das bei der Reaktion gebildete Dimethylammoniumhydrogensulfid im Kühler abschied.

Nach beendeter Reaktion wurde der Ansatz gekühlt, filtriert und das Produkt bei 50°C im Vakuum getrocknet.

Ausbeute 2,4 g (58,4 %), Fp. 122°C.
Die Ausbeute wurde auf Kohlenoxisulfid bzw. Anilin berechnet.

Beispiel 7

N,N-Dimethyl-N'-phenylharnstoff

3,78 g DADTC, 2,33 g Anilin und 50 ml Dimethylformamid wurden gereinigt und unter Rühren auf max. 112°C erhitzt. Nach 240 min war die Reaktion beendet, das Dimethylammonium-

hydrogensulfid hatte sich im Kühler abgeschieden. Die Lösung wurde im Vakuum eingeengt, mit Äther verrieben, filtriert und getrocknet.

Ausbeute: 1,82 g (44,3 %, bezogen auf Kohlenoxisulfid bzw. Anilin), Fp. 123°C.

Beispiel 8

N,N-Dimethyl-N'-phenylharnstoff

7,65 g 98,2%iges DADTC, hergestellt nach Beispiel 5, und 4,65 g Anilin wurden vermischt und unter Rühren 390 min bei max 82°C geschmolzen. Die Schmelze erstarrte gegen Reaktionsende.

Der Feststoff wurde zerkleinert, mit 30 ml Wasser verrieben, filtriert, mit Wasser gewaschen und getrocknet.

Ausbeute: 6,0 g (87,7 %), $N_{gef}$ 17,07 %.

Beispiel 9

N,N-Dimethyl-N'-2-äthylhexylharnstoff

7,51 g DADTC wurden mit 6,47 g 2-Äthylhexylamin und 50 ml Toluol während 220 min am Rückfluß gekocht, wobei die Temperatur von 80 auf 110°C stieg. Die Reaktionsmischung wurde im Vakuum eingeengt. Es blieb ein öliger Rückstand von 10,6 g (Ausbeute 64,6 %) mit einer Reinheit von 61 % zurück, und nach Destillation (152 - 153°C/2-3 mm Hg) ein 96%iges Produkt (gaschromatografisch) in einer Ausbeute von 50 %.

Beispiel 10

N,N-Dimethyl-N'-n-Butylharnstoff

7,51 g DADTC wurden mit 3,66 g n-Butylamin und 50 ml Toluol 3 h unter Rühren am Rückfluß gekocht, wobei die Rückflußtemperatur wieder von 80 auf 110°C anstieg. Die Reaktionslösung wurde im Vakuum eingeengt, der sirupöse Rückstand (6,7 g entsprechend 84,6 % Ausbeute) mit einer Reinheit von 91 % wurde durch Vakuumdestilliation (132 bis 135°C /2-3 mm Hg) gereinigt.

Beispiel 11a

N,N'-Diphenylharnstoff

3,28 g Phenyldimethylharnstoff und 25 ml Anilin wurden unter Einleiten von Kohlenoxisulfid in ca. 30 min auf 200°C erhitzt und zum Rückflußkochen gebracht, nach 150 min abgekühlt und filtriert.

Ausbeute: 4,8 g

$N_{ber}$ 13,20 $N_{gef}$ 13,64

Beispiel 11b

N,N'-Diphenylharnstoff

4,1 g Phenyl-dimethylharnstoff und 2,33 g Anilin wurden in 25 ml Dekalin im Ölbad bei 180 - 190°C 1 h lang erhitzt. Das Reaktionsprodukt wurde nach dem Abkühlen filtriert, mit Äther gewaschen und getrocknet.

Ausbeute: 4,8 g (90 %)

$N_{ber}$ 13,20 $N_{gef}$ 14,21

Das Produkt enthielt noch Ausgangsharnstoff und konnte durch Umkristallisieren aus Äthanol gereinigt werden. Schmelzpunkt: 238 °C

$N_{ber}$ 13,20    $N_{gef}$ 13,46

Beispiel 12

N,N'-Di-(4-chlorphenyl-)harnstoff

3,97 g p-Chlorphenyldimethylharnstoff, 2,55 g p-Chloranilin und 25 ml Dekalin wurden unter Einleiten von Kohlenoxisulfid (7 g) im Verlauf von ca. 20 min auf 180°C erhitzt und anschließend 3 h am Rückfluß gekocht. Nach ca. 35 min zeigten sich im Kühler Dimethylammoniumsulfidabscheidungen, nach 45 min fiel das Reaktionsprodukt aus. Der Ansatz wurde abgekühlt, mit Äther versetzt und filtriert.

Ausbeute: 5,3 g (93,5 %)

$N_{ber}$ 9,96    $N_{gef}$ 10,45
$Cl_{ber}$ 25,22    $Cl_{gef}$ 24,10

Beispiel 13

N-3,4-Dichlorphenyl-N',N'-dichlorphenylharnstoff

4,67 g N-3,4-Dichlorphenyl-N,N'-dimethylharnstoff, 3,24 g 3,4-Dichloranilin wurden bei gleichzeitigem Einleiten von Kohlenoxisulfid (ca. 5 g) in 25 ml Dekalin innerhalb 30 min zum Sieden erhitzt (210°C) und schließlich 2 h am Rückfluß gekocht. Die Reaktionsmischung wurde nach dem Abkühlen filtriert, das Produkt mit Äther gewaschen und bei 100°C getrocknet.

Ausbeute: 2,45 g (35 %)

$N_{ber}$ 8,00    $N_{gef}$ 8,27
$Cl_{ber}$ 40,51    $Cl_{gef}$ 39,60

Beispiel 14

N,N-Dimethyl-N'-2,6-diisopropylphenylharnstoff

3,92 g DADTC (96 %ig) und 4,66 g 2,6-Di-i-propylanilin (95%ig) wurden in 50 ml Toluol bis zur beginnenden Abscheidung des Produktes (etwa 40 min) am Rückfluß gekocht. Nach der Gesamtreaktionszeit von 260 min - die Reaktionstemperatur beträgt am Ende 102°C - wurde die Reaktionsmischung abgekühlt, filtriert und der Niederschlag bei 50°C im Vakuum getrocknet.

Ausbeute: 2,5 g (41 %)
$N_{ber}$ 11,28 $N_{gef}$ 11,08

Beispiel 15

N,N-Dimethyl-N'-2-methylphenylharnstoff

2,68 g o-Toluidin und 3,92 g DADTC (96 %ig) wurden innerhalb 15 min auf 84°C erhitzt, nach insgesamt 315 min Kochzeit abgekühlt, das Produkt isoliert und getrocknet.

Ausbeute: 2,3 g (50 %)
$N_{ber}$ 15,72 $N_{gef}$ 15,33

Beispiel 16

N,N-Dimethyl-N-furfurylharnstoff

3,92 g DADTC (96%ig) und 2,43 Furfurylamin wurden in 50 ml Toluol 310 min am Rückfluß gekocht. Nach dem Abkühlen wurde von wenig Ungelöstem abfiltriert, das Filtrat im Vakuum eingedampft, der Rückstand mit Äther verrieben und filtriert.

Ausbeute: 3,16 g (74,7 %)
Fp: 91°C
$N_{ber}$ 16,56 $N_{gef}$ 16,56

0001251

## Patentansprüche

1. Verfahren zur Herstellung substituierter Harnstoffe aus Kohlenoxisulfid und Aminen, dadurch gekennzeichnet, daß man Harnstoffderivate der allgemeinen Formel:

$$R_1R_2N - \underset{\underset{O}{\|}}{C} - NHR_3$$

darstellt, wobei $R_1$, $R_2$ und $R_3$ gleiche oder verschiedene niedere Alkylgruppen, die gegebenenfalls durch weitere Reste wie Äther- oder Arylgruppen, einen heterocyclischen Rest oder Chlor substituiert sein können, bedeuten, $R_1$ und $R_3$ jeweils eine Alkylgruppe und $R_2$ Wasserstoff darstellt oder $R_1$ und $R_2$ Alkylgruppen sind, die gegebenenfalls durch Wasserstoff ersetzt sein können, wenn $R_3$ eine Arylgruppe, gegebenenfalls durch eine Hydroxy-, Chlor-, Methoxy- oder Alkylgruppe substituiert, ist.

2. Verfahren nach Anspruch 1, dadurch gekennzeichnet, daß man die Reaktionspartner ohne Anwendung von erhöhtem Druck zur Reaktion bringt.

3. Verfahren nach Anspruch 1, dadurch gekennzeichnet, daß man in einem Lösungsmittel arbeitet.

4. Verfahren nach Anspruch 1 und 2, dadurch gekennzeichnet, daß man die Reaktion in der Schmelze durchführt.

5. Verfahren nach Anspruch 1 bis 4, dadurch gekennzeichnet, daß die Reaktionstemperatur etwa -5 bis etwa +200°C beträgt.

6. Verfahren nach Anspruch 1 bis 5, dadurch gekennzeichnet, daß man die Reaktion als Eintopfverfahren durchführt.

7. Verfahren nach Anspruch 1, 2, 3, 5 und 6, dadurch gekennzeichnet, daß man das Filtrat des vorhergehenden Ansatzes als Reaktionsmedium für die folgende Umsetzung verwendet.

Europäisches Patentamt

# EUROPÄISCHER RECHERCHENBERICHT

0001251

Nummer der Anmeldung
EP 78 10 0864

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | GB - A - 1 073 464 (SALZGITTER)<br>* Ansprüche 1-3, 14-17; Seite 4, Beispiele 1,2 *<br>-- | 1-7 |
| X | US - A - 2 655 534 (SEARLE)<br>* Ansprüche 4-6, 10,11; Spalte 4, Beispiel 1; Tabelle *<br>-- | 1-7 |
| A | BE - A - 663 137 (SIGNAL OIL)<br>* Zusammenfassungen 1,2,14,15 *<br>-- | 1 |
| A | FR - A - 1 153 567 (MONSANTO)<br>* Zusammenfassungen 1-11 *<br>-- | 1 |
| A | GB - A - 818 864 (MONSANTO)<br>* Ansprüche 1-24 *<br>-- | 1 |
| DA | CHEMISCHE BERICHTE, 83, (1950) Verlag Chemie GmbH, Weinheim, HAGELLOCH "Umsetzungen von Kohlenoxysulfid mit Aminen", Seiten 258-261<br>* Seiten 260-261; Versuche *<br>-- | 1 |
| A | US - A - 2 857 430 (APPLEGATH et al.)<br>* Ansprüche 1-15; Spalte 6, Beispiel 22 *<br>-- ./. | 1 |

KLASSIFIKATION DER ANMELDUNG (Int.Cl.²)

C 07 C 126/00

RECHERCHIERTE SACHGEBIETE (Int. Cl.²)

C 07 C 127/00
126/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 23-11-1978 | DE ROY |

EPA form 1503.1 06.78

Europäisches Patentamt

# EUROPÄISCHER RECHERCHENBERICHT

Nummer der Anmeldung
EP 78 10 0864
-2-

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| DA | DE - B - 1 232 572 (KALKSTICKSTOFF WERKE)(In der Anmeldung angeführt)<br>* Patentanspruch 1 *<br>-- | | |
| A | US - A - 3 293 293 (DREIER et al.)<br>* Patentanspruch 1 *<br>---- | | |
| | | | RECHERCHIERTE SACHGEBIETE (Int. Cl.²) |